(19) Europäisches Patentamt
European Patent Office
Office européen des brevets

(11) Numéro de publication: **0 239 481**
**A1**

## (12) DEMANDE DE BREVET EUROPEEN

(21) Numéro de dépôt: 87400616.6

(22) Date de dépôt: 19.03.87

(51) Int. Cl.⁴: **A 61 K 9/22,** A 61 K 9/26, A 61 K 31/52

(30) Priorité: **19.03.86 FR 8603932**

(43) Date de publication de la demande: **30.09.87** **Bulletin 87/40**

(84) Etats contractants désignés: **AT BE CH DE ES FR GB GR IT LI LU NL SE**

(71) Demandeur: **Laboratoires DOMS, 4, rue Ficatier, F-92400 Courbevoie (FR)**

(72) Inventeur: **Paris, Laurence, 4, rue Schimper, F-67000 Strasbourg (FR)**
Inventeur: **Stamm, André, 6, rue des Olives Grisheim près de Molsheim, F-67210 Obernai (FR)**

(74) Mandataire: **Ahner, Francis et al, CABINET REGIMBEAU 26, avenue Kléber, F-75116 Paris (FR)**

(54) **Comprimé-retard de théophylline à base de P.V.C., et son procédé de préparation.**

(57) L'invention concerne un comprimé pharmaceutique constitué par une matrice plastique inerte permettant une libération contrôlée d'une dose thérapeutique unitaire de théophylline pendant un intervalle de temps d'environ 8 heures, caractérisé en ce qu'il contient:
- une quantité thérapeutique efficace d'environ 300 à 1000 mg de théophylline anhydre;
- une quantité de P.V.C. d'environ 5 à 15% en poids, par rapport au poids total du comprimé;
- une quantité d'agent lubrifiant interne hydrophobe jusqu'à environ 2% en poids, par rapport au poids total du comprimé.

EP 0 239 481 A1

ACTORUM AG

0239481

1

# COMPRIME-RETARD DE THEOPHYLLINE A BASE DE P.V.C., ET SON PROCEDE DE PREPARATION.

La présente invention concerne de nouveaux comprimés de théophylline constitués par une matrice plastique inerte de chlorure de polyvinyle (P.V.C.) permettant une libération contrôlée du principe actif pendant un intervalle de temps de l'ordre de 6 à 10 heures.

La théophylline ou diméthyl-1,3 xanthine, présente un effet broncho-dilatateur par action sur les muscles lisses des bronches. Il est reconnu que son activité apparaît à partir de concentrations plasmatiques de l'ordre de 0,005 mg/ml et que cette activité est optimale avec des concentrations de l'ordre de 0,015 mg/ml.

La théophylline présente encore d'autres propriétés:
- action sur le centre respiratoire par accélération du rythme des respirations, ainsi qu'une activité sur la pression artérielle respiratoire par vasodilatation locale ;
- facilitation du transport muco-ciliaire par augmentation de la fréquence de battement des cils, et provoque une réduction des oedèmes pulmonaires par une modification de la perméabilité vasculaire ;
- inhibition de la dégranulation des mastocytes, réduisant ainsi les réactions d'hypersensibilité ;
- activité au niveau de l'appareil cardio-vasculaire se caractérisant essentiellement par une vasodilatation périphérique et une augmentation de l'intensité et de la durée des contractions cardiaques , et
- activité au niveau de tous les muscles lisses, estomac, utérus, uretère et sur le rein, conduisant à un effet diurétique très net.

Au niveau toxicologique, les effets secondaires induits par la théophylline apparaissent à partir de concentrations plasmatiques de l'ordre de 0,020 mg/ml. La faible différence entre la concentration thérapeutique efficace et la concentration toxique font de la théophylline un principe actif à marge thérapeutique étroite, nécessitant des précautions particulières lors de l'administration de ce médicament.

La pharmacocinétique de la théophylline présente des variations intra- et inter-individuelles très importantes conduisant sur le plan de la posologie à des prescriptions de l'ordre de 1 200 à 2 000 mg par jour afin de maintenir une concentration plasmatique efficace de l'ordre de 0,012 mg/ml.

Par voie intraveineuse, la dose efficace moyenne est de l'ordre de 400 mg. La dose d'entretien étant fonction de l'âge et de l'état pathologique du sujet.

L'administration orale classique de telles doses, par petites fractions (100-200 mg) à intervalles réguliers oblige le malade à prendre le médicament 6 à 10 fois par jour, sans pour cela que le nycthémère soit totalement couvert. Il existe un risque potentiel d'effets secondaires en cas de non respect de l'intervalle de temps entre les prises du médicament.

Pour diminuer les effets secondaires et le nombre de prises quotidiennes, de nombreuses formes à libération prolongée ont été mises au point, telles que des microgranules, comprimés ou non, des comprimés-matrices à base d'acétylphtalate de cellulose, d'hydroxypropylméthylcellulose ou de résines acryliques. La vitesse de libération du principe actif à partir de ces systèmes est très variable en fonction de la nature de l'excipient, en raison du comportement de ces excipients au contact des milieux digestifs.

Dosées à 300 mg maximum en théophylline, aucune des formes à action prolongée de théophylline actuellement existantes ne conduit à un profil cinétique de libération satisfaisant, à savoir une concentration efficace sur un intervalle d'environ 8 heures. Par conséquent, ces formes à action prolongée de la technique antérieure ne permettent pas la réduction du nombre de prises quotidiennes par rapport aux formes classiques.

L'étude des courbes de pharmacocinétique obtenues avec ces systèmes montre qu'un effet efficace, mais non immédiat, peut être obtenu avec une première prise, en doublant la dose classique, c'est-à-dire en administrant 600 mg.

Sous forme de microgranules, ce dosage pour la voie orale ne peut être raisonnablement envisagé, en raison de la quantité d'excipients nécessaire, et du volume final trop important qu'occuperait la préparation.

La forme comprimé-matrice est plus adaptée à ce dosage à condition de pouvoir limiter le nombre et/ou la quantité d'excipients utilisés.

C'est pourquoi la présente invention concerne un comprimé pharmaceutique constitué par une matrice plastique inerte permettant une libération contrôlée d'une dose thérapeutique unitaire de théophylline pendant un intervalle de temps d'environ 8 heures, caractérisé en ce qu'il contient :

. une quantité thérapeutique efficace d'environ 300 à 1000 mg de théophylline anhydre ;

. une quantité de P.V.C. d'environ 5 à 15 % en poids, par rapport au poids total du comprimé ;

. une quantité d'agent lubrifiant interne hydrophobe d'environ jusqu'à 2% en poids,par rapport au poids total du comprimé.

La théophylline, utilisée à titre de principe actif dans les comprimés-matrice selon la présente invention, peut être sous diverses formes chimiques pharmaceutiquement acceptables et thérapeutiquement actives. De préférence, selon la présente invention, la théophylline utilisée est une base anhydre choisie pour ses caractéristiques physico-chimiques conformes à la Pharmacopée Européenne.

La quantité de théophylline utilisée dans les matrices varie de 300 à 1 000 mg. Elle est, de préférence selon la présente invention, et en raison de besoins thérapeutiques réels de l'adulte de 600 mg.

Les excipients choisis à titre de composants matriciels des comprimés selon la présente invention comprennent du chlorure de polyvinyle (P.V.C.), et un agent lubrifiant interne hydrophobe.

Le P.V.C. est une matière plastique qui fait office, pour les comprimés selon la présente invention, de composant matriciel principal présentant de nombreux avantages. En effet, la littérature indique que le P.V.C. de qualité pharmaceutique, est inerte vis-à-vis du milieu digestif environnant. Par ailleurs, il est dépourvu de tout additif (plastifiants, anti-oxydants, stabilisants thermiques), et sa teneur résiduelle maximale en chlorure de vinyle est fixée à 2 ppm, faisant de cette matière plastique un produit totalement atoxique.

L'utilisation du P.V.C. en tant qu'agent matriciel a déjà été envisagée dans la technique antérieure, mais toujours à des taux soit très importants, de l'ordre de 40% à 95 % en poids par rapport au poids du comprimé, soit à des taux ne permettant pas un profil de libération satisfaisant.

Ainsi, dans le cadre de la présente invention, il est montré que, de manière surprenante et sans que la littérature antérieure n'ait permis de le prévoir, il est possible de réaliser des comprimés - retard fortement dosés en théophylline avec de très faibles proportions en P.V.C., de l'ordre de 5 à 15 % en poids par rapport au poids des comprimés, et sans qu'il soit nécessaire d'ajouter des adjuvants sauf éventuellement un lubrifiant.

Différents P.V.C. pharmaceutiquement acceptables peuvent être utilisés. Dans la pratique, un P.V.C. extrêmement pur diffusé sous le nom de marque PEVIKON® PE 737 P a été utilisé de façon satisfaisante.

Un autre avantage du P.V.C. utilisé dans les comprimés selon la présente invention, est de permettre la réduction considérable de la quantité d'agent lubrifiant interne habituellement nécessaire pour supprimer le grippage occasionné par la théophylline lors de la fabrication des comprimés. Ainsi, la Demanderesse s'est aperçue que, de façon surprenante, une quantité de 0 à 2 % en poids d'agent lubrifiant interne par rapport au poids des comprimés de théophylline, est amplement suffisante dans le but précité. L'agent lubrifiant particulièrement préféré selon l'invention est le stéarate de magnésium. Pour le stéarate de magnésium, ce pourcentage varie de O à 1 %, en fonction de la concavité des poinçons utilisés au cours de la compression.

Les comprimés selon la présente invention, peuvent être préparés par des techniques classiques, connues de l'homme de l'art, à savoir celles qui comprennent une granulation du principe actif, le séchage des granulés obtenus, la pulvérisation des granulés secs puis la compression proprement dite à l'aide d'une pastilleuse rotative ou alternative. Mais de préférence, selon la présente invention, le constituant principal de la matrice, à savoir, le P.V.C., est aussi granulé et ce, parallèlement à la granulation de la théophylline.

Ces deux granulations sont effectuées de préférence par voie humide, et ce, à l'aide de solvants judicieusement choisis.

Le choix de ces solvants repose sur les critères de toxicité, de quantité de liquide nécessaire à la granulation, de pourcentage de particules de taille souhaitée, de friabilité et de porosité des granulés.

De nombreux solvants de granulation sont possibles mais,en fonction des paramètres de choix décrits ci-dessus, l'alcool éthylique a été retenu pour la granulation de la théophylline. Ainsi, par exemple en granulant sur une grille dont l'ouverture de maille est d'approximativement 0,630 mm, ce solvant permet d'obtenir entre environ 80 et 95 % de granulés de théophylline ayant une taille moyenne comprise entre environ 0,5 et 1 mm pour un volume optimal de liquide de l'ordre d'environ 28 à 36 % (v/p).

Pour le P.V.C., l'utilisation du chlorure de méthylène est possible, mais pour des raisons de sécurité au niveau de la fabrication industrielle, on préfère utiliser un mélange binaire de chlorure de méthylène et d'alcool éthylique. Ainsi, par exemple, en granulant le P.V.C. sur une grille dont l'ouverture de maille est d'approximativement 0,630 mm, le chlorure de méthylène, associé à de l'alcool éthylique, de préférence en proportion volumique égale, permet d'aboutir à environ 60 à 70% de granulés de P.V.C. d'une taille moyenne comprise entre 0,5 et 1,0 mm. Ces résultats ont été obtenus pour un volume de mélange de solvants situé entre environ 140 et 150 pour cent (v/p).

Ainsi, les granulations effectuées dans les conditions mentionnées ci-dessus, conduisent à une taille moyenne de granulés comprise entre environ 0,5 et 1 mm pour environ 80 à 95 % des granulés de théophylline et pour environ 60 à 70 % des granulés de P.V.C.

La friabilité des granulés de P.V.C. obtenus est un caractère important car l'invention repose en partie sur cette propriété. La friabilité peut être exprimée par le rapport de friabilité (F) (poids de produit granulé resté intact après agitation mécanique dans des conditions normalisées, rapporté au poids initial). Ce rapport de friabilité (F) est compris entre environ 0,6 et 0,8 dans les conditions opératoires de mesure retenues (Mélangeur TURBULA (R)-W.Bacchoffen-Bâle-Suisse-temps d'agitation de l'ordre de 10 à 20 minutes-vitesse de rotation de 90 t/mn).

Cette friabilité des granulés de P.V.C. permet au cours du mélange des composants granulés, non seulement une lubrification primaire de la masse, mais aussi la formation d'un film hydrophobe discontinu autour des granulés de théophylline par libération de fines particules de P.V.C. de taille inférieure à 0,015 mm et fixation de ces particules sur les granulés de théophylline.

L'obtention d'un tel phénomène a été étudiée aussi bien au niveau de l'homogénéité du mélange, que de la compression de la masse obtenue, mettant ainsi en évidence un optimum, que ce soit dans le temps d'agitation ou dans la vitesse du mélange. Par exemple, sur de petites charges, et avec un mélangeur dit "infini" (TURBULA(R))-W.Bachoffen-Bâle-Suisse), la vitesse de mélange doit être comprise entre environ 25 et 35 t/min pour un temps de mélange (exprimé en minutes) correspondant environ au 1/3 de la masse mise en jeu.

Enfin, l'étude du profil de dissolution de la théophylline en fonction du pH et de la composition du milieu permet de s'assurer de l'efficacité thérapeutique des comprimés selon la présente invention.

Cette étude de dissolution se fait selon la méthode de la "palette tournante" décrite dans la note propharmacopée "Essai de dissolution" (Bull. Ord. Pharm., 1980, mars, n° 79, 402-405), en utilisant comme milieu de dissolution une solution tampon de pH 7,5 répondant à la composition suivante :

$PO_4H_2K$ ............................... 0,87 g
Eau distillée q.s.p. .............. 25 ml
Ajouter :
NaOH 0,2 N ...................... 190 ml
Eau distillée ................... 700 ml
Ajuster au pH 7,5 avec :
HCl N ........................... q.s.
Ajuster le volume à 1 litre.

En 5 heures 30, la dissolution d'un comprimé de théophylline pur obtenu à partir de la théophylline base anhydre retenue, et ceci quel que soit son poids, est totale. Cette dissolution est accélérée par la présence de sels biliaires dans le milieu de dissolution.

Ainsi, il s'est avéré que la matrice et les quantités relatives de chaque constituant du comprimé de la présente invention permet de libérer in vitro un pourcentage de théophylline compris entre environ 10 et 15 % au cours de la première heure.

La particularité de cette formule veut que le pourcentage de théophylline libéré au bout de 8 heures soit sensiblement de 90 à 100% en présence de sels biliaires en concentration physiologique de 8 g/l. Elle n'est que d'environ 50 à 60 % en milieu tampon pH 7,5, sans sels biliaires.

Ainsi, au cours de la compression, la structure obtenue à partir des granulés dont le rapport de fiabilité (F) est compris entre 0,6 et 0,8, est maintenue, conduisant à la formation d'une matrice plastique de texture extrêmement fine, à partir de laquelle la cinétique de libération de la théophylline est régie par l'équation modifiée d'HIGUCHI :

$$Q = A \times \left[ D.K \; (2-K.Cs) \; t^{1/2} \right]$$

où Q représente la quantité libérée au temps t

A représente la quantité de substance présente dans la matrice exprimée par unité de volume (g/cm3), D représente le coefficient de diffusion, K représente le volume spécifique du principe actif, Cs représente la concentration à saturation.

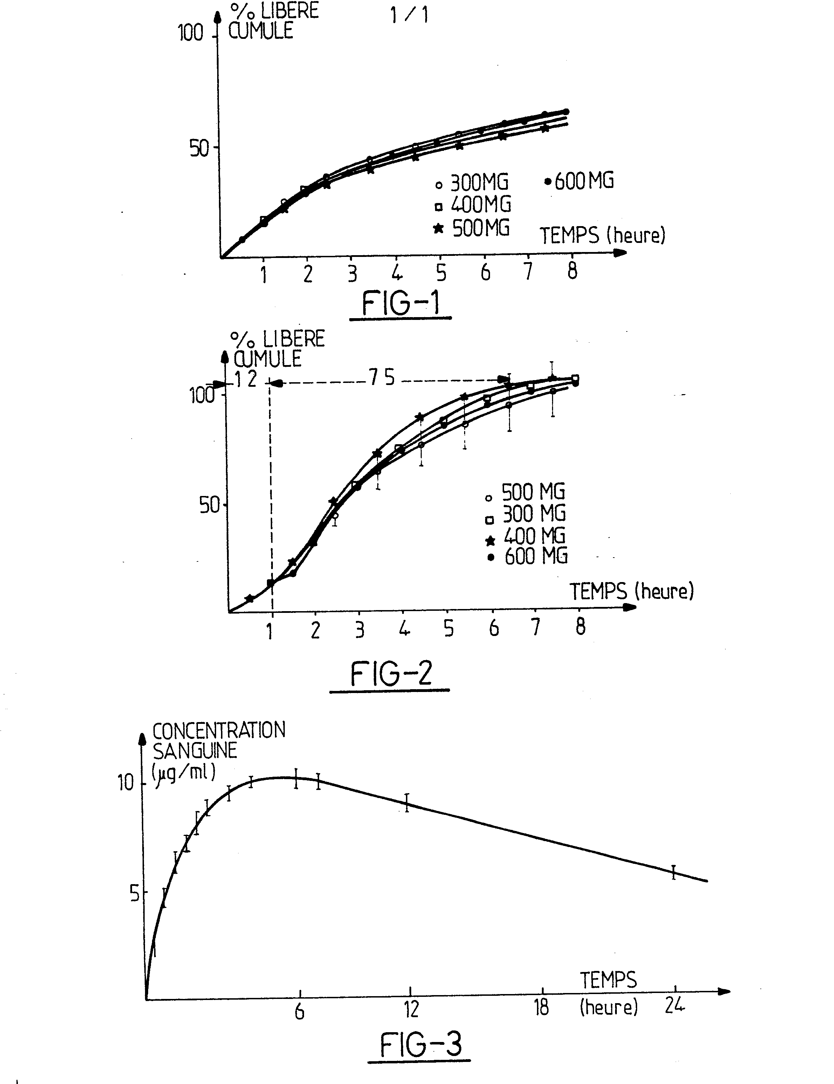

Les études de dissolution effectuées ont permis d'établir les courbes de cinétique de libération de la théophylline, représentées sur les figures 1, 2 et 3.

Possédant ainsi une bonne maîtrise de la formulation pharmaceutique, il est apparu judicieux d'envisager la réalisation d'une forme sécable permettant un ajustement de la posologie en fonction des besoins du malade. Cet objectif est facile à atteindre du fait de l'originalité de la formulation faisant l'objet de la présente invention.

En effet, la structure interne de ces comprimés est suffisamment homogène pour permettre l'obtention de deux portions possédant non seulement un poids équivalent, mais aussi une cinétique de libération de la théophylline de profils identiques entre eux et par rapport au comprimé entier, comme le témoignent les essais réalisés in vitro.

La fabrication de ces comprimés sécables a été réalisée avec des poinçons plats ou bombés : aucune influence de la forme géométrique des comprimés n'est apparue sur la cinétique de libération de la théophylline.

La quantité en théophylline des comprimés, selon la présente invention, est, de préférence, comprise entre 300 et 1 000 mg par comprimé.

A titre d'exemples, les comprimés ayant les compositions suivantes, ont été réalisés :

| | | | |
|---|---|---|---|
| THEOPHYLLINE ANHYDRE (mg) | 300,0 | 400,0 | 500,0 |
| P.V.C. (mg) | 30,0 | 40,0 | 50,0 |
| STEARATE DE MAGNESIUM (mg) | 3,3 | 4,4 | 5,5 |
| | 333,3 | 444,4 | 555,5 |

| | | | | | |
|---|---|---|---|---|---|
| THEOPHYLLINE ANHYDRE (mg) | 600,0 | 700,0 | 800,0 | 900,0 | 1000,0 |
| P.V.C. (mg) | 60,0 | 70,0 | 80,0 | 90,0 | 100,0 |
| STEARATE DE MAGNESIUM (mg) | 6,6 | 7,7 | 8,8 | 9,9 | 11,0 |
| | 666,6 | 777,7 | 888,8 | 999,9 | 1111,0 |

Les exemples et figures ci-après permettent d'illustrer la présente invention, sans pour autant la limiter.

La figure 1 représente l'influence de la teneur en théophylline sur la libération du principe actif à partir des matrices contenant 9,07 % de P.V.C.

La figure 2 représente l'influence des sels biliaires sur la reproductibilité de la formulation.

La figure 3 représente une estimation du profil plasmatique à partir des concentrations salivaires pour des comprimés dosés à 600 mg de théophylline.

EXEMPLE 1 :

Caractéristiques du P.V.C. utilisé : PEVIKON® PE 737P

1.1. Description générale

PEVIKON® PE 737 P est un P.V.C. extrêmement pur, lavé très soigneusement, du type émulsion, avec un haut poids moléculaire et une fine granulométrie. La résine est une poudre blanche finement divisée sans goût ni odeur. Elle est non toxique et physiologiquement inerte. La résine est insoluble dans l'eau, les hydroxydes alcalins et les acides minéraux, soluble dans la cyclohexanone et la tétrahydrofurane.

Ce produit est notamment approuvé par le Swedish National Board of social Welfare et par le Food and Drug Directorate of Canada pour la fabrication de comprimés.

Au niveau de la teneur en chlorure de vinyl monomère résiduel, la législation suédoise précise qu'elle doit être inférieure à 2 ppm, ce qui est aussi la spécification du PEVIKON©PE 737 P.

La détermination du monomère résiduel se fait de la façon suivante :
. Chromatographie en phase gazeuse avec détecteur à ionisation de flamme.
. Colonne d'acier inox de 1/8" de diamètre intérieur et 2 m de longueur, garnissage Poropac R 80 - 100 mesh.
. Un échantillon de 0,5 g est introduit dans un flacon en verre de 20 ml puis chauffé à 100°C pendant 90 minutes, puis refroidi à température ambiante.

On fait l'analyse de l'espace de tête avec 1 ml de la phase gazeuse.

1.2. Données techniques
Propriétés testées selon ISO 1060 (voir chapitre 2.2. du PEVIKON manual)

| | |
|---|---|
| Valeur en K (0,5 % dans cyclohexanone) | approx.73 |
| nombre de viscosité ISO 174 | 136 ± 7,5 ml/g |
| densité de charge ISO 60 | non spécifié |
| analyse sur tamis < 0,063 mm ISO 1624 | > 99,0 % |
| matières volatiles ISO 1269 | < 0,3 % |
| extrait de méthanol ASTM D-2222 | approx. 0,4 % |
| contenu en sulfate ($SO_4^{--}$) | < 95 mg/kg |

monomère de chlorure de vinyle                    < 2 mg/kg
Designation selon ISO 1060 : PVCeP 136 3.


1.3. Valeurs typiques

valeur en K (0,5 % dans cyclohexanone)        73
poids moléculaire (nombre moyen)          70 000
poids moléculaire (poids moyen)          250 000
densité de charge                            0,33 g/ml
matières volatiles                           0,10 %
distribution des tailles des particules :

                              > 10 µm        70 %
                              > 25 µm        25 %
                              > 100 µm        0,05 %
solubilité dans l'eau (100°C, 2 jours)       0,23 %
teneur en sulfate (SO$_4^{--}$)              85 mg/kg

metaux :

                              Zn             0,3 mg/kg
                              Mn             0,3 mg/kg
                              Fe             0,6 mg/kg
                              Cr             0,3 mg/kg

émulsifiant (non toxique)                    0,4 %


EXEMPLE 2

Etude de l'influence du volume de solvant de granulation et du mode de séchage des granulés sur les propriétés des comprimés selon la présente invention

En plus du choix de la matrice et des quantités relatives de chaque constituant du comprimé, il est apparu au cours des diverses expérimentations réalisées, qu'un certain nombre de paramètres de mise en oeuvre du procédé de fabrication des comprimés pouvaient également avoir une influence sur les propriétés physiques et thérapeutiques du comprimé de théophylline.

Deux paramètres importants se sont avérés influencer de façon significative les propriétés physiques des granulés ainsi que la libération de la théophylline à partir des matrices plastiques à savoir :

- le volume de solvant de granulation, et
- le mode de séchage des granulés.

Le volume de liquide influe directement sur la taille des granulés obtenus. Sur une même classe granulométrique, par exemple 0,5-1,0 mm, des variations importantes peuvent être observées au niveau du diamètre moyen des particules.

Le mode de séchage joue un rôle important dans la structure finale du granulé.

Ceci est d'autant plus important dans le cas des matières plastiques.

Ces constatations sont valables à la fois pour la théophylline et pour le P.V.C.

Pour la théophylline, l'étude de ces deux paramètres a permis de mettre en évidence qu'une variation d'environ 8 % (v/p) de la quantité de solvant de granulation autour de la valeur optimale, provoque une modification de la taille des particules de l'ordre de 0,060 mm induisant une variation du pourcentage de théophylline libérée d'environ 10 à 20 %.

Le séchage à l'air ambiant de cette matière est néfaste à une bonne cohésion des comprimés, occasionnant une accélération du pourcentage libéré de la théophylline de l'ordre de 10 à 50 %. La stabilisation de la libération est observée à partir d'un temps de séchage à l'étuve à environ 110 degrés de l'ordre de 20 à 30 minutes.

L'étude menée dans le cadre du P.V.C. indique qu'une variation du volume optimal de solvant d'environ 10 % (v/p) conduit à une modification de l'ordre de 0,200 mm de la taille moyenne des particules ainsi qu'à une variation de pourcentage de théophylline libéré située entre environ 10 et 30 %.

Lorsque la taille agumente sous l'effet de l'accroissement du solvant de granulation, la friabilité est réduite, entraînant une accélération du processus de dissolution.

Au niveau des propriétés physiques des granulés de P.V.C., la proportion de solvant chloré dans le liquide de granulation est importante. Une réduction d'environ 50 % de la quantité de solvant chloré dans le mélange de solvants servant à la granulation augmente la friabilité dans de fortes proportions (le rapport de friabilité passe d'environ 0,7 à environ 0,1).

Contrairement à la théophylline, le séchage à l'étuve à environ 110°C est néfaste au P.V.C. Cette matière prend une structure élastique empêchant la cohésion du comprimé. Dans ces conditions, la vitesse de libération est multipliée par environ 2, quel que soit le temps de séjour à l'étuve. Ce mode de séchage est cependant applicable si la température de l'étuve est inférieure à environ 90°C, température de transition vitreuse du P.V.C. (passage de l'état plastique à l'état élastique).

Il est à signaler que contrairement à de nombreux systèmes thérapeutiques matriciels, la matrice faisant l'objet de la présente invention, ne voit pas sa structure affectée par la force de compression mise en jeu. En conséquence, la vitesse de libération reste constante quelle que soit la pression mise en jeu au cours de la compression.

De nombreuses autres techniques de granulation ont été étudiées, telles qu'une granulation globale des constituants ou une dissolution de la matière plastique. Celles-ci n'ont pas conduit à un profil de cinétique de libération satisfaisant, ou se sont avérées insensibles aux sels biliaires

En conclusion, après avoir maîtrisé, à la suite des études fondamentales, la libération in vitro de la théophylline à partir de la matrice, les premiers résultats obtenus in vivo ont rapidement confirmé l'intérêt de cette forme pharmaceutique.

L'étude in vivo des comprimés dosés à 600 mg de théophylline et présentant une libération de 90 à 100 % de théophylline sur un intervalle de temps de 8 heures en présence de sels biliaires, assurent après une dose unique chez trois volontaires sains une théophyllinémie de 0,010 mg/ml dès la quatrième heure. Cette concentration est maintenue pendant cinq heures.

En tenant compte des paramètres moyens de pharmacocinétique des volontaires sains, et en se fixant pour concentration minimale 0,010 mg/ml et maximale 0,015 mg/ml, le nombre de prises par jour se trouve réduit à 1. Ainsi, contrairement aux formes dosées à 300 mg, l'état d'équilibre peut être atteint au bout de 1 à 2 jours, à raison d'une prise par jour au lieu de 3 à 4 jours avec 2 prises par jour pour les formes actuellement commercialisées.

REVENDICATIONS

1. Comprimé pharmaceutique constitué par une matrice plastique inerte permettant une libération contrôlée d'une dose thérapeutique unitaire de théophylline pendant un intervalle de temps d'environ 8 heures, caractérisé en ce qu'il contient :

. une quantité thérapeutique efficace d'environ 300 à 1000 mg de théophylline anhydre ;

. une quantité de P.V.C. d'environ 5 à 15 % en poids, par rapport au poids total du comprimé ;

. une quantité d'agent lubrifiant interne hydrophobe d'environ jusqu'à 2 % en poids, par rapport au poids total du comprimé.

2. Comprimé selon la revendication 1, caractérisé en ce qu'il est obtenu par un procédé comprenant la granulation humide séparée de la théophylline et du P.V.C., la compression de granulés de théophylline et de granulés de P.V.C. lesdits granulés ayant été séchés, et additionnés d'un agent lubrifiant interne hydrophobe.

3. Comprimé selon la revendication 2, caractérisé en ce que le solvant utilisé pour la granulation du P.V.C. comprend un mélange binaire de chlorure de méthylène et d'alcool éthylique dans des proportions volumiques sensiblement identiques, et en ce que le volume dudit solvant utilisé est sensiblement compris entre 140 et 150 % par rapport au poids de la substance à granuler.

4. Comprimé selon la revendication 2, caractérisé en ce que le solvant utilisé pour la granulation de la théophylline comprend de l'alcool éthylique et en ce que le volume dudit solvant utilisé est sensiblement compris entre 28 et 36 % par rapport au poids de la substance à granuler.

5 . Comprimé selon la revendication 2, caractérisé en ce que environ 80 à 95 % des granulés de théophylline ont une taille comprise entre environ 0,5 et 1 mm.

6 . Comprimé selon la revendication 2, caractérisé en ce que environ 60 à 70 % des granulés de PVC ont une taille comprise entre environ 0,5 et 1 mm.

7 . Comprimé selon les revendications 2 et 3., caractérisé en ce que le rapport de friabilité (F) des granulés de PVC est compris entre environ 0,6 et 0,8.

8 . Comprimé selon la revendication 1, caractérisé en ce que l'agent lubrifiant interne hydrophobe utilisé est le stéarate de magnésium.

9 . Comprimé selon les revendications 1 à 8 , caractérisé en ce que le pourcentage de théophylline libéré in vitro en présence de sels biliaires sur un intervalle de temps de 8 heures est sensiblement compris entre 90 et 100 %.

10 . Comprimé selon les revendications 1 à 9 , caractérisé en ce qu'il est sécable.

11·. Comprimé selon les revendications 1 à 10, caractérisé en ce qu'il présente la composition suivante exprimée en mg :

Théophylline anhydre ........................ 600,0
P.V.C. pharmaceutiquement acceptable ........ 60,0
Stéarate de magnésium ....................... 6,6
------
666,6

12. Procédé de préparation des comprimés selon les revendications 1 à 11, caractérisé en ce qu'on granule par voie humide la théophylline à l'aide d'un solvant dont le volume est sensiblement compris entre 28 et 36 % par rapport au poids de la théophylline, ledit solvant comprenant de l'alcool éthylique, on sèche les granulés de théophylline à l'étuve à une température d'environ 110°C pendant environ 20 à 30 minutes, on granule par voie humide le P.V.C. à l'aide d'un solvant dont le volume est sensiblement compris entre 140 et 150 % par rapport au poids du PVC, ledit solvant comprenant de l'alcool éthylique et du chlorure de méthylène en proportions volumiques égales, on sèche les granulés de P.V.C. à une température inférieure à environ 90°C, on mélange les granulés de théophylline et de P.V.C., on additionne éventuellement du stéarate de magnésium, on mélange à nouveau et on effectue la mise en forme des comprimés à l'aide d'une pastilleuse rotative ou alternative.

0239481

1 / 1

FIG-1

FIG-2

FIG-3

Office européen
des brevets

**RAPPORT DE RECHERCHE EUROPEENNE**

0239481

Numéro de la demande

EP  87 40 0616

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | CHEMICAL ABSTRACTS, vol. 103, no. 12, 23 septembre 1985, pages 314-315, résumé no. 92742q, Columbus, Ohio, US; L. PARIS et al.: "Study on plastic matrixes of theophylline. I. Effects of various factors on formulation of matrixes", & EXPO. - CONGR, INT. TECHNOL. PHARM., 3rd 1983, 2, 143-53 <br> * Résumé * | 1-12 | A 61 K     9/22 <br> A 61 K     9/26 <br> A 61 K    31/52 |
| | --- | | |
| Y | CHEMICAL ABSTRACTS, vol. 103, no. 12, 23 septembre 1985, page 315, résumé no. 92743r, Columbus, Ohio, US; L. PARIS et al.: "Study of plastic matrixes of theophylline. 2. Study of release as a function of tablet formation conditions", & EXPO. - CONGR. INT. TECHNOL. PHARM., 3rd 1983, 2, 154-64 <br> * Résumé * | 1-12 | |
| | --- | | DOMAINES TECHNIQUES RECHERCHES (Int. Cl.4) |
| Y | EP-A-0 134 290  (VEREX LABORATORIES, INC.) <br> * En entier * | 1-12 | A 61 K |
| | --- | | |
| Y | GB-A-2 078 518  (BRISTOL-MYERS) <br> * Page 2, lignes 21-41; page 4, tableau * | 1-12 | |
| | ---                    -/- | | |

Le présent rapport de recherche a été établi pour toutes les revendications

| Lieu de la recherche | Date d'achèvement de la recherche | Examinateur |
|---|---|---|
| LA HAYE | 11-06-1987 | BENZ K.F. |

0239481

Office européen
des brevets

**RAPPORT DE RECHERCHE EUROPEENNE**

Numéro de la demande

EP 87 40 0616

| | **DOCUMENTS CONSIDERES COMME PERTINENTS** | | Page 2 |
|---|---|---|---|
| Catégorie | Citation du document avec indication, en cas de besoin, des parties pertinentes | Revendication concernée | CLASSEMENT DE LA DEMANDE (Int. Cl.4) |
| A | CHEMICAL ABSTRACTS, vol. 95, no. 24, décembre 1981, page 368, no. 209531e, Columbus, Ohio, US; H. FESSI et al.: "Biopharmaceutical study of tablets of plastic matrix type: effect of the solubility of active principle and the nature of polymer on the dissolution rate", & C.-R. - CONGR. EUR. BIOPHARM. PHARMACOCINET, 1st 1981, 1, 475-80 * Résumé * | | |
| | --- | | |
| A | DE-A-2 126 810 (AB HÄSSLE) * Page 1, lignes 1-10; pages 8-9, exemple 3 * | | |
| | ----- | | **DOMAINES TECHNIQUES RECHERCHES (Int. Cl.4)** |

Le présent rapport de recherche a été établi pour toutes les revendications

| Lieu de la recherche | Date d'achèvement de la recherche | Examinateur |
|---|---|---|
| LA HAYE | 11-06-1987 | BENZ K.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82